(19) Europäisches Patentamt
European Patent Office
Office européen des brevets

(11) **EP 2 835 147 B1**

(12) **EUROPEAN PATENT SPECIFICATION**

(45) Date of publication and mention
of the grant of the patent:
**13.11.2019 Bulletin 2019/46**

(51) Int Cl.:
***A61M 37/00*** (2006.01)

(21) Application number: **13771861.5**

(22) Date of filing: **02.04.2013**

(86) International application number:
**PCT/JP2013/060078**

(87) International publication number:
**WO 2013/151044 (10.10.2013 Gazette 2013/41)**

(54) **PUNCTURE DEVICE**

PUNKTIONSVORRICHTUNG

DISPOSITIF DE PONCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.04.2012 JP 2012086415**

(43) Date of publication of application:
**11.02.2015 Bulletin 2015/07**

(73) Proprietor: **Hisamitsu Pharmaceutical Co., Inc. Tosu-shi, Saga 841-0017 (JP)**

(72) Inventors:
• **ARAMI, Shunsuke**
 **Tsukuba-shi**
 **Ibaraki 305-0856 (JP)**
• **OGURA, Makoto**
 **Tsukuba-shi**
 **Ibaraki 305-0856 (JP)**
• **TOKUMOTO, Seiji**
 **Tsukuba-shi**
 **Ibaraki 305-0856 (JP)**

(74) Representative: **Grünecker Patent- und Rechtsanwälte**
 **PartG mbB**
 **Leopoldstraße 4**
 **80802 München (DE)**

(56) References cited:
EP-A1- 2 626 107      EP-A1- 2 937 111
WO-A1-2004/009172      WO-A1-2005/123173
WO-A1-2006/103727      WO-A2-2011/112229
JP-A- 2004 510 534      JP-A- 2006 276 200
JP-A- 2006 500 973      JP-A- 2010 211 890
US-A1- 2011 276 027

## Description

## Technical Field

The present invention relates to a puncture device for transferring an active ingredient into a body through a skin.

## Background Art

There have conventionally been known puncture devices in which a microneedle member equipped with a number of microneedles having their tips coated with a medicament and the like is held by a latch mechanism and the like (see Patent Literatures 1 to 5). The microneedle member held by the puncture device is released, so as to collide with a skin, whereby an active ingredient contained in the medicament and the like transfers into an animal (e.g., human) body.

## Citation List

## Patent Literature

Patent Literature 1: JP 4659332 B
Patent Literature 2: JP 2007-516781 A
Patent Literature 3: WO 2009/107806
Patent Literature 4: WO 00/009184
Patent Literature 5: US 2011/276027 Document WO 2004/009172 A1 is considered to represent the closest prior art and it discloses a puncture device to be used for delivering drugs through a skin, wherein it comprises a miocroneedle member, a slidable piston in a tubular housing and a coil spring that actuates the piston.

## Summary of Invention

## Technical Problem

However, the conventional puncture devices have been large in size. Depending on the medicament and the like, the microneedles may be required to be kept mounted on the skin for several ten minutes after colliding therewith to transfer the active ingredient in to the body adequately. Therefore, the puncture devices have been desired to be made further smaller in size and lighter in weight to make it easy to wear or carry.

It is therefore an object of the present invention to provide a puncture device which can be made smaller in size and lighter in weight, as well as improve the durability of the collision speed rate to the skin.

## Solution to Problem

The puncture device in accordance with the present invention is defined by the features of claim 1.

The puncture device in accordance with the present invention uses a conical coil spring for providing a piston with a biasing force. When compressed, the conical coil spring has a height much smaller than that of typical cylindrical coil springs. Therefore, the puncture device can reduce its own height and attain a lighter weight.

Depending on the kind of medicaments and the like, puncture devices must be held for a long time on the skin after colliding therewith. Even in such a case, the puncture device in accordance with this mode of the present invention made smaller in size and lighter in weight enables its user to wear clothes and move without limitation while keeping the puncture device attached to the skin. The puncture device in accordance with the present invention is small in size and thus is very unlikely to collide with other objects (obstacles) and let the microneedles come out of the skin or break and remain in the skin even when the user behaves freely as such.

The conventional large puncture devices may take time to handle or intimidate users because of large exterior sizes. By contrast, the puncture device in accordance with this mode of the present invention made smaller in size and lighter in weight can easily be handled and greatly reduce the fear that might be felt by the users.

The piston may have a piston body formed with a groove and a microneedle member 30 provided with an engagement piece adapted to engage the groove, while the microneedle member may include a surface constituting the main surface of the piston, the plurality of microneedles projecting from the surface. When attaching the microneedle member to the piston body with an adhesive or the like so as to integrate them together, organic compounds contained in the adhesive may affect the medicament and the like applied to the tips of the microneedles. However, the microneedle member mechanically attached to the piston body with the engagement piece so as to be integrated with the piston body in this case does not affect the medicament and the like and enables them to exhibit their intrinsic effects.

The conical coil spring may be formed by a stainless steel wire, a piano wire, plastics, or a copper wire.

The conical coil spring has a compressed height that is smaller, which allows the puncture device to further reduce its size and weight.

The conical coil spring may have a free height of at least three times a wire diameter thereof. This allows the conical coil spring to provide the piston with a sufficient energy when compressed.

The conical coil spring may have a free height of 1 mm to 100 mm. When the free height of the conical coil spring is less than 1 mm, the puncture device is less likely to provide a sufficient puncture performance. When the free height of the conical coil spring exceeds 100 mm, it tends to be difficult for users to behave while keeping the puncture device attached.

3    EP 2 835 147 B1    4

A metal wire constituting the conical coil spring may be free of overlapping parts as seen in an extending direction of a center line of the conical coil spring. In this case, when a load is applied to the conical coil spring along the extending direction of the center line, the height of the compressed coil spring substantially coincides with its wire diameter. This allows the puncture device to further reduce its size and weight.

The conical coil spring may have both end parts cut flat along a virtual plane orthogonal to the center line of the conical coil spring. This increases contact areas between the conical coil spring and members such as the piston which constitute the puncture device, since both end parts of the conical coil spring come into contact with these members. Therefore, the conical coil spring can be placed stably within the puncture device. The conical coil spring having both end parts cut flat has larger contact areas with members such as the piston than those without such flattening and thus allows the members such as the piston to collide with the skin without substantially tilting them from its advancing direction. Hence, the skin can be pierced more appropriately.

The conical coil spring may have a maximum diameter of 1 mm to 100 mm. When the free height of the conical coil spring is less than 1 mm, the puncture device is less likely to provide a sufficient puncture performance. Since areas which can be considered flat in animal skins are limited, it becomes harder to attach the puncture device stably to the skins when the maximum diameter of the conical coil spring exceeds 100 mm.

The conical coil spring may have a minimum diameter at least 1/1000 but less than 1 times the maximum diameter thereof.

The conical coil spring may have a wire diameter of 0.1 mm to 2 mm.

The conical coil spring may have a load of 10,8 N (1100 gf) to 49 N (5000 gf) under compression.

## Advantageous Effects of Invention

One aspect of the present invention can provide a puncture device which can be made smaller in size and lighter in weight. Another aspect of the present invention is that the collision speed rate to the skin is less affected also after longer storage of the device and at a higher temperature.

References to embodiments throughout the description which are not under the scope of the appended claims merely represent possible exemplary executions and are therefore not part of the present invention.

## Brief Description of Drawings

Fig. 1 is a perspective view of a device in accordance with an embodiment as seen from thereabove;
Fig. 2 is a perspective view of the device in accordance with the embodiment as seen from thereunder;
Fig. 3 is a perspective view illustrating a piston and a microneedle member;
Fig. 4 is a perspective view illustrating a cap;
Figs. 5(a) and 5(b) are sectional views taken along the line V-V of Fig. 2 when the piston is biased and after the piston is actuated, respectively;
Fig. 6 is a perspective view partly illustrating the microneedle member;
Fig. 7 is a sectional view taken along the line VII-VII of Fig. 7;
Fig. 8 is a set of diagrams for explaining an example of methods for coating microneedles;
Fig. 9 is a set of sectional views illustrating conical coil springs;
Fig. 10 is a set of sectional views illustrating examples of nonlinear coil springs;
Fig. 11 is a table listing conditions for carrying out Examples 1 to 27 and Comparative Examples 1 and 2 and results of their evaluations;
Fig. 12 is a chart illustrating the relationship between free height and velocity;
Fig. 13 is a chart illustrating the relationship between free height and load under compression; and
Fig. 14 is a table listing conditions for carrying out Examples 28 to 30 and results of their evaluations.

## Description of Embodiments

[Structure of Puncture Device]

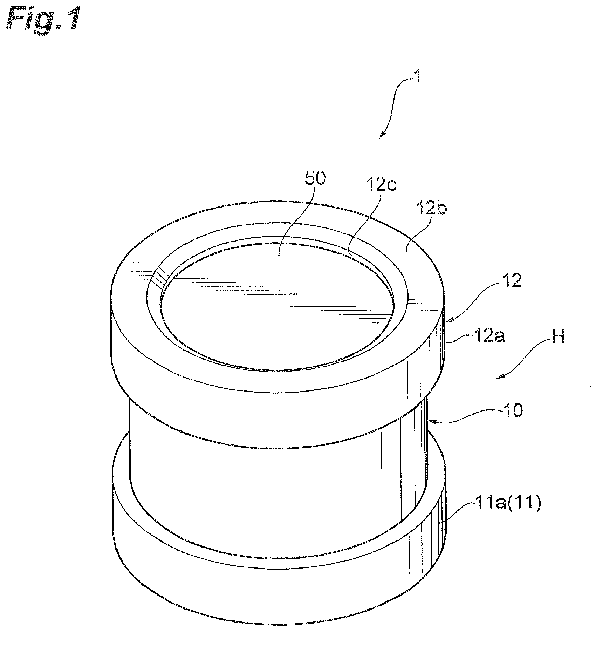

A puncture device 1 in accordance with an embodiment will be explained with reference to Figs. 1 to 10. In the explanation, the same constituents or those having the same functions will be referred to with the same signs while omitting their overlapping descriptions.

The puncture device 1 is a device for transferring an active ingredient such as a medicament into a body of an animal such as a human through a skin thereof. The puncture device 1 comprises a housing H having a cylindrical form, a piston P having a piston body 20 and a microneedle member 30, a conical coil spring 40, and a cap 50.

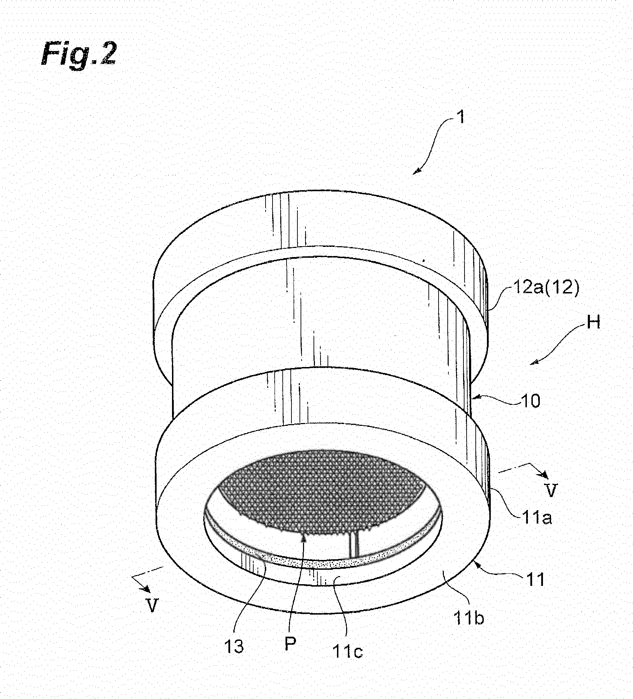

As illustrated in Figs. 1 and 2, the housing H has a tubular body 10 in a cylindrical form and ring members 11, 12 attached to both ends of the housing H, respectively. The housing H has such a strength as to be able to keep a biasing force of the conical coil spring 40 (which will be explained later in detail). Examples of materials for the housing H include not only synthetic and natural resin materials such as ABS resins, polystyrene, polypropylene, and polyacetal (POM), but also silicone, silicon dioxide, ceramics, and metals (stainless steel, titanium, nickel, molybdenum, chromium, cobalt, and the like).

It is preferred for the puncture device 1 to have a form which is easy to hold and apply (stick) microneedles 32 to skins of animals (including humans). There-

3

fore, the tubular body 10 may have a polygonal or rounded outer form. The surface of the tubular body 10 may be dented or stepped. The surface of the tubular body 10 may be roughened by forming fine grooves or a nonslip coating layer thereon. Through holes may be formed in the tubular body 10 in order to reduce its air resistance and weight.

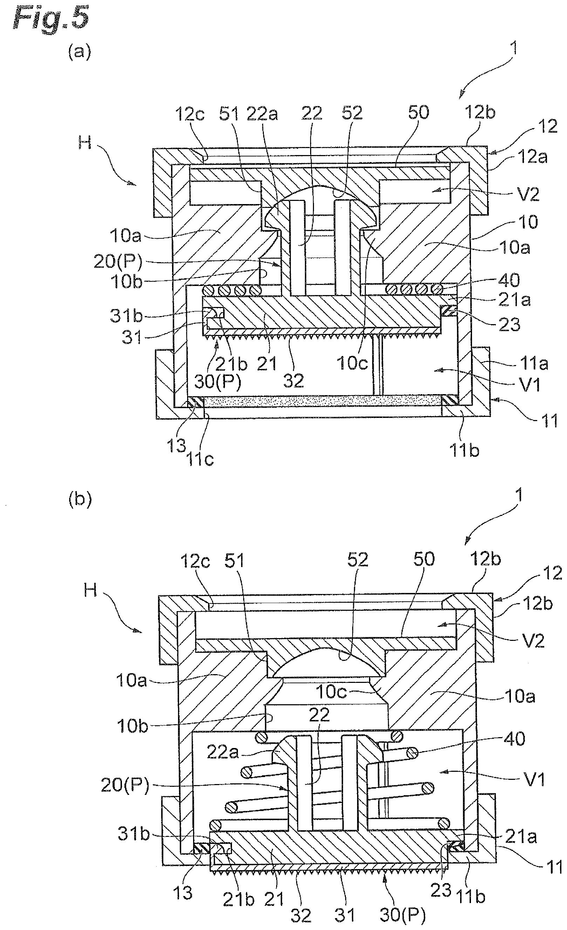

The ring member 11, which is a body separate from the tubular body 10, is attachable to and detachable from the tubular body 10. As illustrated in Figs. 2 and 5, the ring member 11 has a cylindrical side wall part 11a and a bottom wall part 11b extending inward from an end part of the side wall part 11a. The bottom wall part 11b is formed with a circular through hole 11c. The through hole 11c has a diameter smaller than the inner diameter of the tubular body 10.

As illustrated in Figs. 2 and 5, an annular buffer member 13 is attached to the inner side face of the bottom wall part 11b. The buffer member 13 is constructed by an elastic material. Examples of the elastic material include rubber and silicone; in particular, silicone which is hard to deteriorate with time may be used.

The ring member 12, which is a body separate from the tubular body 10, is attachable to and detachable from the tubular body 10. As illustrated in Fig. 1, the ring member 12 has a cylindrical side wall part 12a and a top wall part 12b extending inward from an end part of the side wall part 12a. The top wall part 12b is formed with a circular through hole 12c. The through hole 12c has a diameter smaller than the inner diameter of the tubular body 10.

As illustrated in Fig. 5, a partition wall 10a for partitioning the inside of the tubular body 10 is provided therein on the ring member 12 side. This divides the inside of the tubular body 10 into a space V1 closer to the ring member 11 than is the partition wall 10a and a space V2 closer to the ring member 12 than is the partition wall 10a.

A through hole 10b extending along the direction in which the housing H (tubular body 10) extends is formed at a center part of the partition wall 10a. The through hole 10b has a cylindrical surface and communicates the spaces V1, V2 to each other. An inwardly projecting ring-shaped projection 10c is provided within the through hole 10b. The projection 10c reduces its diameter from the ring member 11 side to the ring member 12 side.

As illustrated in Fig. 2, the piston P is slidably arranged within the space V1 of the housing H (tubular body 10). Specifically, the piston P moves between the ring members 11, 12 within the space V1 along the extending direction of the housing H (tubular body 10).

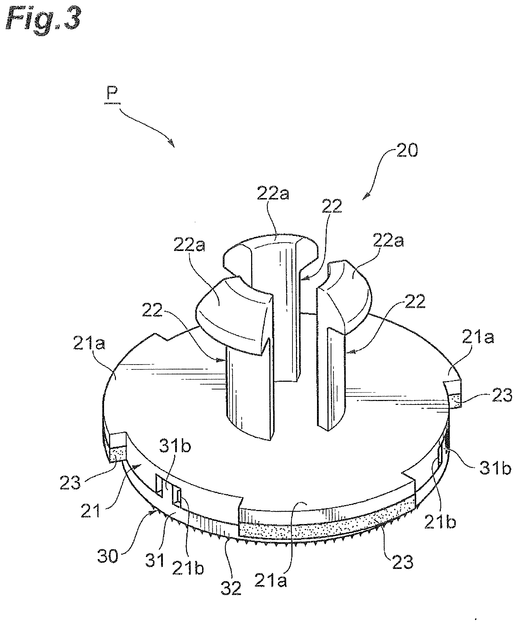

In this embodiment, the piston P is constructed by the piston body 20 and the microneedle member 30 as illustrated in Fig. 3. The piston body 20 has a piston plate 21, three piston rods 22, and a buffer member 23. Through holes may be formed in the piston body 20 in order to reduce its air resistance and weight. The piston body 20 may be made of the same material as with a base 31 and microneedles 32, which will be explained later.

The piston plate 21 is formed like a disk and has a pair of main surfaces. In a state where the piston body 20 is arranged within the housing H (tubular body 10), the pair of main surfaces of the piston plate 21 intersect (are substantially orthogonal to) the sliding direction of the piston body 20 (the extending direction of the housing 10).

The outer periphery of the piston plate 21 is provided with three outwardly projecting planar projections 21a. The projections 21a are arranged at substantially equally spaced intervals along the outer periphery of the piston plate 21. One main surface of each projection 21a is the same as one main surface of the piston plate 21, while the other main surface of the projection 21a faces the other surface of the piston plate 21.

The side face of the piston plate 21 is formed with three grooves 21b (see Figs. 3 and 5). The grooves 21b are arranged at substantially equally spaced intervals along the outer periphery of the piston plate 21.

Three piston rods 22 are erected near the center of one main surface of the piston plate 21. The three piston rods 22 are arranged on the one main surface while being separated from each other at equally spaced intervals about the center of the piston plate 21.

Each piston rod 22 has a tip 22a projecting out toward the outer periphery of the piston plate 21 and functioning as a hook for engaging the projection 10c within the through hole 10b. The tip 22a tapers toward its tip.

The buffer member 23 is attached to the other main surface of the projection 21a. The buffer member 23 is constituted by the same material as with the buffer member 13.

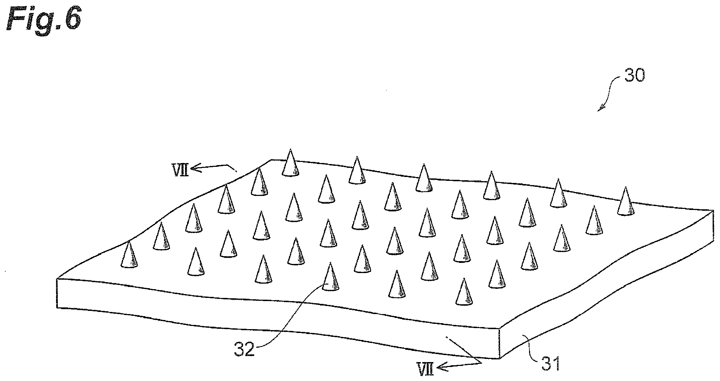

As illustrated in Figs. 5 and 6, the microneedle member 30 has a disk-shaped base 31 and a plurality of microneedles 32. The base 31 is a foundation to support the microneedles 32. The base 31 may have an area of $0.5 \text{ cm}^2$ to $300 \text{ cm}^2$, $1 \text{ cm}^2$ to $100 \text{ cm}^2$, or $1 \text{ cm}^2$ to $50 \text{ cm}^2$. A plurality of bases 31 may be joined together, so as to construct a base having a desired size.

The outer periphery of the base 31 is provided with three projections 31b extending along a direction in which a pair of main surfaces of the base 31 oppose each other (see Figs. 3 and 5). The projections 31b are placed at substantially equally spaced intervals along the outer periphery of the base 31. Each projection 31 has a tip portion projecting toward the center of the base 31 and functioning as a hook for engaging the groove 21b formed in the side face of the piston plate 21. When the projections 31b engage their corresponding grooves 21b, the microneedle member 30 is integrally attached to the other main surface of the piston body 20.

As illustrated in Fig. 6, the microneedles 32 project from a surface of the base 31. In this embodiment, the surface of the base 31 from which the microneedles 32 project constitutes a main surface of the piston P. The microneedle 32 are arranged at substantially equally spaced intervals in a zigzag (staggered) pattern on the surface of the base 31.

The microneedles 32 may have a height (length) of 20 $\mu$m to 700 $\mu$m or 50 $\mu$m to 700 $\mu$m. The height of the microneedles 32 is at least 20 $\mu$m in order to transfer a medicament or the like securely into the body. The height of the microneedles 32 is 700 $\mu$m or less in order for the microneedles 32 to pierce only the cuticle of the skin without reaching the dermis.

Each microneedle 32 is a tapered structure thinning from its base part, which is connected to the base 31, toward its tip part. That is, microneedle 32 is a structure having a needle shape or containing a needle shape. The microneedle 32 may be formed with a pointed tip like a circular cone or polygonal pyramid or without a pointed tip like a truncated circular cone or a truncated polygonal pyramid. When the microneedle 32 has a conical form as illustrated in Fig. 6, the diameter at its base part may be 5 $\mu$m to 250 $\mu$m or 10 $\mu$m to 200 $\mu$m.

When the tip of the microneedle 32 is rounded, the radius of curvature of the tip part may be 2 $\mu$m to 100 $\mu$m or 5 $\mu$m to 30 $\mu$m. When the tip of the microneedle 32 is flat, the flat part may have an area of 20 $\mu$m$^2$ to 600 $\mu$m$^2$ or 50 $\mu$m$^2$ to 250 $\mu$m$^2$.

As for the density of microneedles 32 on the base 31, 1 to 10 microneedles 32 are typically arranged per 1 mm in each line. In general, lateral lines adjacent to each other are separated from each other by a distance substantially equal to the space of the microneedles 32 within the lateral line. The density of microneedles 32 is consequently 100 to 10000 per 1 cm$^2$, but may also be 200 to 5000 per 1 cm$^2$, 300 to 2000 per 1 cm$^2$, or 400 to 850 per 1 cm$^2$.

The base 31 and microneedles 32 may be made of the same material or different materials. All the microneedles 32 may be made of the same material, or those made of different materials may be mixed therewith. Examples of materials for the base 31 and microneedles 32 include silicone, silicon dioxide, ceramics, metals (stainless steel, titanium, nickel, molybdenum, chromium, cobalt, and the like) and synthetic and natural resin materials. When the antigenicity of the base 31 and microneedles 32 and the unit cost of materials are taken into consideration, examples of resin materials include biodegradable polymers such as polylactic acid, polyglycolide, poly(lactic-co-glycolic acid), pullulan, caprolactone, polyurethane, and polyanhydrides, and non-biodegradable polymers such as polycarbonates, polymethacrylic acid, ethylene vinyl acetate, polytetrafluoroethylene, and polyoxymethylene. Also usable are polysaccharides such as hyaluronic acid, sodium hyaluronate, pullulan, dextran, and dextrin, chondroitin sulfate, and cellulose derivatives. Products formed by compounding active ingredients in the above-mentioned biodegradable resins may also be used as materials for the base 31 and/or microneedles 32 in other embodiments.

The material for the microneedles 32 may also be a biodegradable resin such as polylactic acid for fear of breaking on the skin. While polylactic acid encompasses polylactic acid homopolymers such as poly-L-lactide and poly-D-lactide, poly-L/D-lactide copolymers, their mixtures, and the like, any of them may be used. Polylactic acid increases strength with its average molecular weight; one having an average molecular weight of 40000 to 100000 may be used.

Examples of methods for making the base 31 and microneedles 32 include wet etching or dry etching using a silicon substrate, precision machining using a metal or resin (e.g., electric discharge machining, laser machining, dicing, hot embossing, and injection molding), and machine cutting. Such machining integrally forms the base 31 and microneedle 32. An example of methods for hollowing the microneedles 32 is secondarily processing the microneedles 32 with laser machining and the like after making them.

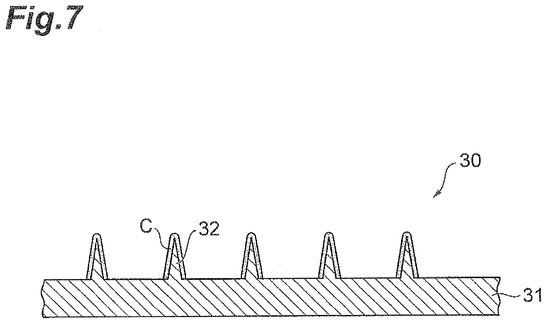

As illustrated in Fig. 7, a coating C of an active ingredient may be applied onto the base 31 and/or microneedles 32. In this embodiment, the coating C is one in which a coating liquid including a polymeric carrier compatible with the active ingredient is anchored to a part or whole of the base 31 and/or microneedles 32. Examples of the polymeric carrier include carboxyvinyl polymers, polyethylene oxide, polyvinylpyrrolidone, polyvinyl alcohol, and cellulose derivatives. By "anchored" is meant that a state where the coating liquid is substantially uniformly attached to an object is maintained. The coating liquid is anchored in a dry state by a known drying method such as air drying, vacuum drying, freeze drying, or their combinations immediately after the coating, but is not always anchored in the dry state after transdermal administration, since it may hold moisture contents which are in equilibrium with the surrounding atmosphere, organic solvents, and the like.

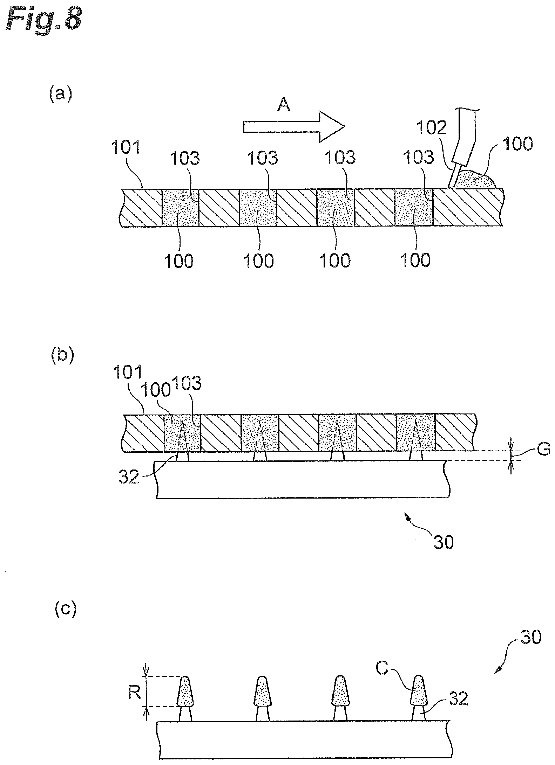

An example of methods for coating the microneedles 32 will be explained with reference to Fig. 8. First, as illustrated in Fig. 8(a), a coating liquid 100 is swept in the direction of arrow A on a masking sheet 101 with a spatula 102, so as to fill openings 103 of the masking sheet 101 with the coating liquid 100. Subsequently, as illustrated in Fig. 8(b), the microneedles 32 are inserted into the openings 103 of the masking sheet 101. Thereafter, as illustrated in Fig. 8(c), the microneedles 32 are pulled out of the openings 103 of the masking sheet 101. This applies the coating C onto the surfaces of the microneedles 32. The coating C is anchored to the microneedles 32 when dried.

A range R of the coating C on the microneedles 32 is adjusted by a gap G (see Fig. 8(b)) or the thickness of the masking sheet 101. The gap G, which is defined by the distance from the base parts of the microneedles 32 to the lower face of the masking sheet 101 (not concerned with the substrate thickness), is set according to the tension of the masking sheet 101 and the height of the microneedles 32. The range of the distance of the gap G may be 0 $\mu$m to 500 $\mu$m. When the distance of the gap G is 0 μm, the microneedles 32 are totally coated. The range R of the coating C varies depending on the height of the microneedles 32 and may be more than 0 μm but not exceeding 500 μm, typically 10 μm to 500 μm, or on the order of 30 μm to 300 μm.

The thickness of the coating C on the base 31 and/or microneedle 32 may be less than 50 μm, less than 25 μm, or 1 μm to 10 μm. In general, the thickness of the coating C is an average of thicknesses measured throughout the surfaces of the microneedles 32 after the drying. The thickness of the coating C can typically be increased by employing a plurality of films of coating carriers, i.e., by repeating the coating step after anchoring the coating carrier.

When coating the base 31 and/or microneedle 32, the temperature and humidity of an environment in which the device is installed may be held constant in order to minimize changes in concentration and physical properties of the medicament caused by evaporation of the solvent from the coating agent. For preventing the solvent from evaporating, a drop in temperature, a rise in humidity, or both may be controlled. The humidity at room temperature without temperature control may be 50% RH to 100% RH, 70% RH to 100% RH, or 90% RH to 100% RH in terms of relative humidity. At 50% RH or thereunder, the solvent evaporates remarkably, thereby changing physical properties of the coating solution. Humidifying schemes, examples of which include vaporization, steaming, and water spraying, are not limited in particular as long as the aimed state of humidity can be secured. For minimizing the volatility of the solvent, highly wettable and humectant water-soluble polymers may be mixed with the coating solution.

The coating agent includes an active ingredient, and distilled water and/or a coating carrier. Examples of the coating carrier include polyethylene oxide, hydroxymethylcellulose, hydroxypropylcellulose, hydroxypropylmethylcellulose, methylcellulose, carboxymethylcellulose, carmellose sodium, dextran, polyethylene glycol, polyvinyl alcohol, polyvinylpyrrolidone, pullulan, chondroitin sulfate, hyaluronic acid, sodium hyaluronate, dextrin, gum arabic, ethanol, isopropanol, methanol, propanol, butanol, propylene glycol, dimethyl sulfoxide, glycerin, N,N-dimethylformamide, polyethylene glycol, benzyl benzoate, sesame oil, soybean oil, lactic acid, benzyl alcohol, polysorbate 80, alpha-thioglycerin, ethylenediamine, N,N-dimethylacetamide, thioglycolic acid, and phenoxyethanol.

Water-soluble carriers compatible (uniformly miscible) with the active ingredient may also be used as the coating carrier. Their specific examples include polyvinylpyrrolidone, polyvinyl alcohol, carboxyvinyl polymers, polyacrylic acid, sodium polyacrylate, polyoxyethylene polyoxypropylene glycol, Pluronic, polyethylene oxide, polyethylene glycol, propylene glycol, glycerin, butylene glycol, polyvinylacetamide, hydroxypropylcellulose, and pullulan. Specific examples among them include carboxyvinyl polymers, polyethylene oxide, polyvinylpyrrolidone, hydroxypropylcellulose, pullulan, propylene glycol, glycerin, and butylene glycol.

The content of the coating carrier in the coating agent may be 0.1% by weight to 70% by weight, 0.1% by weight to 60% by weight, or 1% by weight to 40% by weight. The coating carrier may be required to be viscous to some extent in order to prevent it from dripping, i.e., to have a viscosity on the order of 100 cps to 100000 cps. The viscosity may also be 500 cps to 60000 cps. When the viscosity falls within this range, a desired amount of the coating solution can be applied at once regardless of the material of the microneedles 32. In general, the amount of the coating solution tends to increase as the viscosity is higher.

The liquid composition used for coating the base 31 and/or microneedles 32 is prepared by mixing a biocompatible carrier, a useful active ingredient to be delivered, and, if necessary, any coating adjuvant with a volatile liquid. The volatile liquid may be any of water, dimethyl sulfoxide, dimethylformamide, ethanol, isopropyl alcohol, and their mixtures. Among them, water may be used in particular. The liquid coating solution or suspension may typically have a useful bioactive ingredient concentration of 0.1% by weight to 65% by weight, which may also be 1% by weight to 40% by weight or 10% by weight to 30% by weight. The coating may attain an anchored state. A zwitterionic, amphoteric, cationic, anionic, or nonionic surfactant may be used. For example, Tween 20 and Tween 80, other sorbitan derivatives such as sorbitan laurate, and alkoxylated alcohols such as Laureth-4 may be used. For example, adding the surfactant is also effective in dissolving a larger amount of the active ingredient in the coating carrier.

Other known pharmaceutical adjuvants may be added to the coating as long as they do not detrimentally affect the features of solubility and viscosity required for the coating and characteristics and physical properties of the dried coating.

The active ingredient usable in this embodiment is not limited in particular, but may include all the ingredients used in the medical and cosmetic fields, for example. Examples of active ingredients usable in the medical field include anti-infective drugs such as preventive medicines (antigens), antibiotics, and antivirals; analgesics; pain-killing combination drugs; anesthetics; anorectics; antiarthritics; antiasthmatics; anticonvulsants; antidepressants; antidiuretics; antidiarrheals; antihistamine drugs; anti-inflammatory drugs; antimigraine drugs; motion sickness drugs; antiemetics; antineoplastics; antiparkinsonian drugs; antipruritics; antipsychotics; antipyretics; gastrointestinal and urinary antispasmodics; anticholinergics; sympathomimetics; xanthine derivatives; cardiovascular drugs including calcium channel blockers; beta blockers; beta agonists; antiarrhythmic drugs; antihypertensives; ACE inhibitors; diuretics; general, coronary, peripheral, and cerebral vessel vasodilators; central nervous system stimulants; cough and cold medicines; decongestants; diagnostic drugs; hormones;

hypnotics; immunosuppressants; muscle relaxants; parasympatholytic blockers; parasympatholytic agonists; prostaglandins; proteins; peptides; polypeptides; psychostimulants; sedatives; and tranquilizers.

The above-mentioned antigens as the active agent usable in this embodiment are not limited in particular and may be polynucleotides (DNA and RNA vaccines), peptide antigens, and protein-based vaccines. Their specific examples include those in the forms of polysaccharides, oligosaccharides, lipoproteins, attenuated or killed viruses such as cytomegalovirus, hepatitis B virus, hepatitis C virus, human papilloma virus, rubella virus, and varicella-zoster virus; attenuated or killed bacteria such as pertussis bacteria, Clostridium tetani, Corynebacterium diphtheriae, Group A Streptococcus, Legionella pneumophila, Neisseria meningitidis, Pseudomonas aeruginosa, Streptococcus pneumoniae, Treponema pallidum, and Vibrio cholerae; and their mixtures. The antigens include a number of commercially available vaccines containing antigenic substances, examples of which include influenza vaccine, Lyme disease vaccine, rabies vaccine, measles vaccine, epidemic parotitis vaccine, varicella vaccine, smallpox vaccine, hepatitis vaccine, pertussis vaccine, and diphtheria vaccine, as well as antigens used in vaccine therapies such as those for cancer, arteriosclerosis, nervous diseases, and Alzheimer's disease. In addition, the antigens may be allergenic substances having antigenicity (sensitizing property), which include various metals and chemical substances. For example, in allergy tests and treatments for clarifying antigens of atopic dermatitis, dust, house dust such as inactivated mites, various kinds of pollen, and the like may be used. Antigens recognized by inflammatory T cells related to T cell-mediated autoimmune diseases or symptoms are also included.

The active ingredient used in this embodiment may include plant preparations such as extracts or tinctures for treating local skin diseases. Examples of the extracts or tinctures include oak bark extracts, walnut extracts, arnica tinctures, witch hazel extracts, Plantago lanceolata extracts, pansy extracts, thyme or sage extracts; St. John's wort tinctures, golden glow extracts, chamomile flower extracts, or calendula tinctures; and, for instance, birch leaf extracts, nettle extracts, coltsfoot extracts, comfrey tinctures, horsetail extracts, aloe extracts, Aesculus turbinata and Ruscus aculeatus extracts, and arnica, calendula, and chili pepper extracts, for taking care of heavily tired and damaged skins.

The active ingredient usable outside of the pharmaceutical field is selected from the group consisting of antioxidants, free radical scavengers, humectants, depigmentation agents, fat controllers, UV-reflecting agents, wetting agents, antimicrobial agents, antiallergic drugs, anti-acne drugs, anti-aging drugs, anti-wrinkle drugs, bactericides, anti-baldness drugs, hair growth promoters, hair growth inhibitors, anti-dandruff agents, keratolytics, soft drinks, peptides, polypeptides, proteins, deodorants, antiperspirants, skin softeners, skin moisturizer solutions, softening agents, hair conditioners, hair softeners, hair moisturizers, tanning agents, skin-whitening agents, antifungal agents, depilatories, analgesic drugs for external use, counterirritants, drugs for hemorrhoids, insecticides, therapeutic drugs for poison ivy rash, therapeutic drugs for poison sumac rash, therapeutic drugs for burns, anti-diaper rash drugs, drugs for heat rash, skin lotions, vitamins, amino acids, amino acid derivatives, herb extracts, retinoids, flavonoids, sense markers, skin conditioners, hair lighteners, chelating agents, cellular turnover enhancers, coloring agents, sunscreens, revitalizers, water absorbers, sebum absorbers, and their mixtures.

As the active ingredient usable in this embodiment, amino acids include not only salts, esters, and acyl derivatives thereof but also amino acids obtained by hydrolyzing various proteins. Examples of such amino acid drugs include amphoteric amino acids such as alkylamidoalkylamines, stearyl acetyl glutamate, capryloyl silk amino acids, and capryloyl collagen amino acids; capryloyl keratin amino acids; capryloyl pea amino acids; cocodimonium hydroxypropyl silk amino acids; corn gluten amino acids; cysteine; glutamic acid; glycine; hair keratin amino acids; hair amino acids such as aspartic acid, threonine, serine, glutamic acid, proline, glycine, alanine, half-cystine, valine, methionine, isoleucine, leucine, tyrosine, phenylalanine, cysteic acid, lysine, histidine, arginine, cysteine, tryptophan, and citrulline; lysine; silk amino acids; wheat amino acids; and their mixtures.

As the active ingredient usable in this embodiment, each of the peptides, polypeptides, and proteins includes a polymer having a long chain composed of at least about 10 carbon atoms and a high molecular weight of at least 1000, for example, which is formed by self-condensation of amino acids. Examples of such proteins include collagen; deoxyribonucleases; iodized corn proteins; keratin; milk proteins; proteases; serum proteins; silk; sweet almond proteins; wheat germ proteins; wheat proteins; alpha and beta helices of wheat proteins or keratin proteins; and hair proteins such as intermediate filament proteins, high-sulfur-content proteins, ultrahigh-sulfur-content proteins, intermediate-filament-associated proteins, high-tyrosine proteins, high-glycine/tyrosine proteins, trichohyalin, and their mixtures

Examples of anti-wrinkle ingredients usable in this embodiment include hyaluronic acid, sodium hyaluronate, retinol (vitamin A), silybin peptides (HTC collagen, palmitoyl penta, Peptide 3, and Argireline), amino acids, hydroxyproline, tocopheryl retinoate, ursolic acid, vitamin C derivatives, coenzyme Q10, astaxanthin, fullerene, polyphenols, alpha lipoic acid, soybean extracts, pullulan, active isoflavone, sugars, polysaccharides, glycerin, and glycerin derivatives. However, the anti-wrinkle ingredients are not limited to the above, but may be mixed.

Sodium hyaluronate is promising as a coating carrier and an anti-wrinkle ingredient. In particular, low-molecular-weight sodium hyaluronate having a molecular weight on the order of 50000 to 110000 is highly adherent to the microneedle member 30 than is high-molecular-weight sodium hyaluronate.

Preferred examples of vitamins usable in this embodiment include vitamin B complexes; vitamins A, C, D, E, and K and their derivatives, e.g., vitamin A palmitate, including thiamine, nicotinic acid, biotin, pantothenic acid, choline, riboflavin, vitamin B6, vitamin B12, pyridoxine, inositol, and carnitine; provitamins such as panthenol (provitamin B5) and panthenol triacetate; and their mixtures.

Examples of antimicrobial agents usable in this embodiment include bacitracin, erythromycin, neomycin, tetracycline, chlortetracycline, benzethonium chloride, phenol, and their mixtures.

Examples of skin softeners and skin moisturizers usable in this embodiment include mineral oils, lanolin, vegetable oils, isostearyl isostearate, glyceryl laurate, methyl gluceth-10, methyl gluceth-20, chitosan, and their mixtures.

Examples of hair conditioners usable in this embodiment include not only lipophilic compounds such as cetyl alcohol, stearyl alcohol, hydrogenated polydecene, and their mixtures, but also quaternary compounds such as behenamidopropyl PG-dimonium chloride, tricetyl ammonium chloride, dihydrogenated tallowamidoethyl hydroxyethylmonium methosulfate, and their mixtures.

Examples of sunscreens usable in this embodiment include butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, methoxycinnamic acid diethanolamine, glycerin aminobenzoate, titanium dioxide, zinc oxide, oxybenzone, Padimate O, red petrolatum, and their mixtures. A preferred tanning agent usable in this embodiment is dihydroxyacetone.

Examples of skin-whitening agents usable in this embodiment include hydroquinone and its derivatives, catechol and its derivatives, ascorbic acid and its derivatives, ellagic acid and its derivatives, kojic acid and its derivatives, tranexamic acid and its derivatives, resorcinol derivatives, placenta extracts, arbutin, oil-soluble licorice extracts, and their mixtures.

Examples of anti-inflammatory analgesics usable in this embodiment include acetaminophen, methyl salicylate, monoglycol salicylate, aspirin, mefenamic acid, flufenamic acid, indomethacin, diclofenac, alclofenac, diclofenac sodium, ibuprofen, ketoprofen, naproxen, pranoprofen, fenoprofen, sulindac, fenclofenac, clidanac, flurbiprofen, fentiazac, bufexamac, piroxicam, phenylbutazone, oxyphenbutazone, clofezone, pentazocine, mepirizole, and tiaramide hydrochloride. Examples of steroidal anti-inflammatory analgesics usable in this embodiment include hydrocortisone, prednisolone, dexamethasone, triamcinolone acetonide, fluocinolone acetonide, hydrocortisone acetate, prednisolone acetate, methylprednisolone, dexamethasone acetate, betamethasone, betamethasone valerate, flumethasone, fluorometholone, and beclomethasone dipropionate.

Examples of antihistaminic drugs usable in this embodiment include diphenhydramine hydrochloride, diphenhydramine salicylate, diphenhydramine, chlorpheniramine hydrochloride, chlorpheniramine maleate, isothipendyl hydrochloride, tripelennamine hydrochloride, promethazine hydrochloride, and methdilazine hydrochloride. Examples of local anesthetics usable in this embodiment include dibucaine hydrochloride, dibucaine, lidocaine hydrochloride, lidocaine, benzocaine, p-buthylaminobenzoic acid 2-(diethylamino) ethyl ester hydrochloride, procaine hydrochloride, tetracaine, tetracaine hydrochloride, chloroprocaine hydrochloride, oxyprocaine hydrochloride, mepivacaine, cocaine hydrochloride, piperocaine hydrochloride, dyclonine, and dyclonine hydrochloride.

Examples of bactericides and disinfectants usable in this embodiment include thimerosal, phenol, thymol, benzalkonium chloride, benzethonium chloride, chlorhexidine, povidone iodine, cetylpyridinium chloride, eugenol, and trimethylammonium bromide. Examples of vasoconstrictors usable in this embodiment include naphazoline nitrate, tetrahydrozoline hydrochloride, oxymetazoline hydrochloride, phenylephrine hydrochloride, and tramazoline hydrochloride. Examples of hemostats usable in this embodiment include thrombin, phytonadione, protamine sulfate, aminocaproic acid, tranexamic acid, carbazochrome, carbazochrome sodium sulfonate, rutin, and hesperidin.

Examples of chemotherapeutic drugs usable in this embodiment include sulfamine, sulfathiazole, sulfadiazine, homosulfamine, sulfisoxazole, sulfisomidine, sulfamethizole, and nitrofurazone. Examples of antibiotics usable in this embodiment include penicillin, methicillin, oxacilline, cephalothin, cephalodine, erythromycin, lincomycin, tetracycline, chlorotetracycline, oxytetracycline, methacycline, chloramphenicol, kanamycin, streptomycin, gentamycin, bacitracin, and cycloserine.

Examples of antiviral drugs usable in this embodiment include protease inhibitors, thymidine kinase inhibitors, sugar or glycoprotein synthesis inhibitors, constituent protein synthesis inhibitors, adherence and adsorption inhibitors, and nucleoside analogs such as acyclovir, penciclovir, valaciclovir, and ganciclovir.

Examples of hair growth stimulants or hair restorers usable in this embodiment include minoxidil, carpronium chloride, pentadecanoic acid glycerides, tocopherol acetate, piroctone olamine, glycyrrhizic acid, isopropylmethylphenol, hinokitiol, Swertia japonica extracts, ceramides and their precursors, nicotinic acid amide, and chili pepper tinctures.

Examples of cosmetically active ingredients usable in this embodiment include D-alpha tocopherol, DL-alpha tocopherol, D-alpha-tocopheryl acetate, DL-alpha-tocopheryl acetate, ascorbyl palmitate, vitamin F and vitamin F glycerides, vitamin D, vitamin D2, vitamin D3, retinol, retinol esters, retinyl palmitate, retinyl propionate, beta carotene, coenzyme Q10, D-panthenol, farnesol, farnesyl acetate; jojoba oil and blackcurrant oil abundantly contained in essential fatty acids; 5-n-octanoyl salicylic acid and its esters, salicylic acid and its esters; alkyl esters of alpha hydroxy acids such as citric acid, lactic acid, and glycolic acid; asiatic acid, madecassic acid, asiaticoside, total extracts of Centella asiatica, beta glycyrrhetinic acid, alpha bisabolol, ceramides such as 2-oleoylamino-1,3-octadecane; phytantriol, marine-derived phospholipids abundantly contained in poly-unsaturated essential fatty acids, ethoxyquin; rosemary extracts, balm extracts, quercetin, dry microalgal extracts, anti-inflammatory drugs such as steroidal anti-inflammatory drugs, and biochemical stimulants such as hormones or fats and/or compounds attributed to the synthesis of proteins.

Vitamin C usable in this embodiment promotes collagen (connective tissue) synthesis, lipid (fat) and carbohydrate metabolisms, and neurotransmitter synthesis. Vitamin C is also essential for optimally maintaining the immune system. Vitamin C is toxic to a wide range of cancer cells, such as melanoma in particular. Tyrosine oxidase, which catalyzes aerobic activities of tyrosine changing into melanin and other pigments, is inhibited from acting in the presence of vitamin C. Vitamin C has been found effective in catalyzing immune responses to infections with many viruses and bacteria. In addition to many applications mentioned above, vitamin C is essential for synthesizing collagen and treating external wounds. This embodiment may include not only vitamins C and E, but also combinations of other ingredients such as humectants, collagen synthesis promoters, and facial scrubs.

Examples of skin conditioner ingredients in this embodiment include mineral oils, petrolatum, vegetable oils (e.g., soybean oil and maleated soybean oil), dimethicone, dimethicone copolyol, cationic monomers and polymers (e.g., guar hydroxypropyltrimonium chloride and distearyl dimethyl ammonium chloride), and their mixtures. Examples of humectants include polyols such as sorbitol, glycerin, propylene glycol, ethylene glycol, poly(ethylene glycol), polypropylene glycol, 1,3-butanediol, hexylene glycol, isoprene glycol, xylitol, fructose, and their mixtures.

These active ingredients may be used either singly or in combination of two or more and in any form of inorganic and organic salts as long as they are pharmaceutically acceptable. The active ingredients are basically incorporated in the coating carrier, but may be supplied later via through holes formed in the base 31 without including the active ingredients into the coating carrier. The active ingredients may directly be applied to the skin before putting the microneedle member 30 onto the same part of the skin. In this case, the skin stretching effect and the ODT (occlusive dressing therapy) effect on the skin can promote infiltration of the active ingredients into the skin.

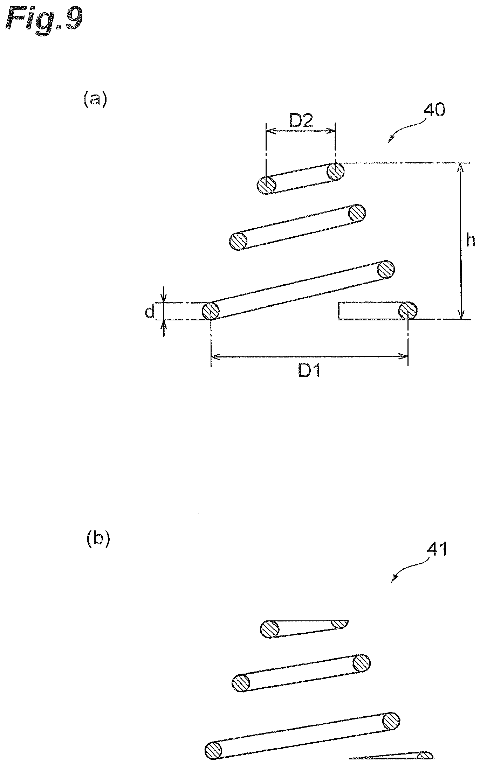

The conical coil spring 40 is arranged between one main surface of the piston plate 21 and the partition wall 10a, having the piston rods 22 inserted at the center of the conical coil spring 40. As illustrated in Figs. 5 and 9(a), the conical coil spring 40 is formed by spirally winding a metal wire having a circular cross section such that it appears as a cone when seen from a side. In this embodiment, the conical coil spring 40 has no overlapping part when seen in its center line direction. Examples of the metal wire include stainless steel wires, piano wires (iron wires), and copper wires. Among them, the stainless steel wires are very hard to corrode in particular.

In this embodiment, the smaller and larger diameter sides of the conical coil spring 40 abut against the partition wall 10a and piston plate 21 side, respectively. The minimum diameter of the conical coil spring 40 is greater than the diameter of the through hole 10b. This prevents the conical coil spring 40 from shifting toward the space V2 through the through hole 10b. The maximum diameter of the conical coil spring 40 is smaller than the diameter of the piston plate 21. Therefore, the conical coil spring 40 can securely bias the piston plate 21.

Parameters for the energy of the piston P actuated by the biasing force of the conical coil spring 40 include the modulus of transverse elasticity, wire diameter (d in Fig. 9(a)), maximum diameter (D1 in Fig. 9(a)), minimum diameter (D2 in Fig. 9(a)), total number of turns, weight of the conical coil spring 40, weight of the piston P (piston body 20 and microneedle member 30), free height (h in Fig. 9(a)), solid height, pitch angle, and pitch.

The modulus of transverse elasticity is determined by the material of the conical coil spring 40. The modulus of transverse elasticity is 68500 $N/mm^2$ in a stainless steel wire, 78500 $N/mm^2$ in a piano wire (iron wire), and $3.9 \times 10^4$ $N/mm^2$ to $4.4 \times 10^4$ $N/mm^2$ in a copper wire. The wire diameter d of the conical coil spring may be 0.01 mm to 2 mm, 0.1 mm to 1.5 mm, or 0.3 mm to 1.3 mm. The wire diameter d of the metal wire constituting the conical coil spring 40 may be fixed or vary like a taper coil spring from one end to the other end.

It is sufficient for the maximum diameter D1 to be at least 4 times the wire diameter. The maximum diameter D1 may be 1 mm to 100 mm, 1 mm to 50 mm, or 5 mm to 30 mm. When the maximum diameter D1 is less than 1 mm, the puncture device 1 is less likely to provide a sufficient puncture performance. Since areas which can be considered flat in animal skins are limited, it becomes harder to attach the puncture device 1 stably to the skins when the maximum diameter D1 exceeds 100 mm.

The minimum diameter D2 may be at least 1/1000 but less than 1 times, 1/100 to 2/3 times, or 1/10 to 1/2 times the maximum diameter D1. For example, the minimum diameter D2 may be 1 mm to 100 mm, 1 mm to 50 mm, 1 mm to 20 mm, or 1 mm to 10 mm. In particular, the minimum diameter D2 may be 0.33 to 0.38 times or 0.34 to 0.37 times the maximum diameter D1.

The total number of turns may be 1 to 100, 1 to 10, or 2 to 5. The weight of the conical coil spring 40 may be 0.01 g to 10 g, 0.1 g to 5 g, or 0.1 g to 3 g. The weight of the piston P (piston body 20 and microneedle member 30) may be 0.1 g to 20.0 g, 0.2 g to 10.0 g, or 0.3 g to 0.6 g.

The free height is preferably at least 3 times the wire diameter. For example, the free height may be 1 mm to 100 mm, 2 mm to 20 mm, or 2 mm to 10 mm. When the free height is less than 1 mm, the puncture device 1 is less likely to provide a sufficient puncture performance. When the free height exceeds 100 mm, it tends to be difficult for users to behave while keeping the puncture device 1 attached.

The conical coil spring 40 may be heat-treated before being used in the puncture device 1. This can enhance the life of the conical coil spring 40. That is, the heat treatment can restrain the conical coil spring 40 from fatiguing (worsening its mechanical properties) when compressed. The heat treatment time may be at least 1 min, at least 10 min, or at least 20 min, for example.

The conical coil spring 40 may have a load of 10,8 N (1100 gf) to 49 N (5000 gf) under compression.

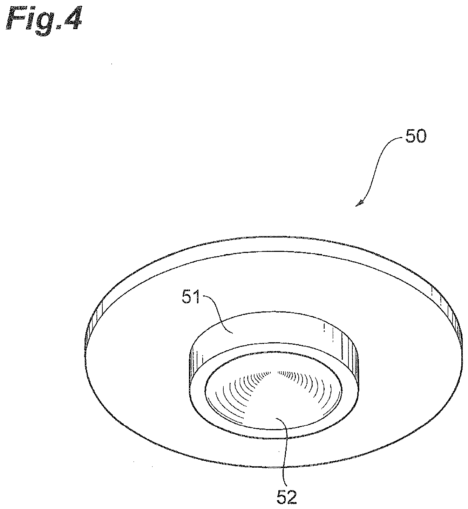

As illustrated in Fig. 4, the cap 50 is formed like a disk. The diameter of the cap 50 is substantially the same as or slightly smaller than the inner diameter of the housing 11. Therefore, the cap 50 is contained in the space V2 of the housing H (tubular body 10) and is prevented by the ring member 12 attached to the tubular body 10 from getting out of the space V2. The cap 50 may be made of the same material as with the housing H. Through holes may be formed in the cap 50 in order to reduce its air resistance and weight.

A cylindrical projection 51 is provided in a center part of one main surface of the cap 50. The diameter of the projection 51 is substantially the same as or slightly smaller than that of the through hole 10b. Therefore, the projection 51 is guided along the through hole 10b. The projection 51 is formed with a mortar-shaped depression 52 which reduces its diameter toward the cap 50.

[Method for Manufacturing Puncture Device]

A method for manufacturing the puncture device 1 will now be explained. First, the above-mentioned components (tubular body 10, ring members 11, 12, piston body 20, microneedle member 30, conical coil spring 40, and cap 50) of the puncture device 1 are prepared. The microneedles 32 of the prepared microneedle member 30 have been coated with the coating C beforehand.

Next, the conical coil spring 40 is attached to the piston P. Specifically, the piston P is stood such that the microneedle member 30 and the piston body 20 are located on the lower and upper sides, respectively. Then, the conical coil spring 40 is mounted on one main surface of the piston body 20 with the larger and smaller diameter sides being located on the lower and upper sides, respectively, while the piston rods 22 are inserted therethrough. This allows the conical coil spring 40 to erect stably when being attached to the piston P, whereby the puncture device 1 is easier to manufacture.

Subsequently, the piston P having the conical coil spring 40 attached thereto is pushed into the tubular body 10 from the space V1 side. Here, since a biasing force occurs in compressed conical coil spring 40, the piston P is pushed under a load which surpasses the biasing force. The tips 22a of the piston rods 22 pass through the through hole 10b while being deflected toward the center of the through hole 10b (the center of the piston plate 21) by the projection 10c of the tubular body 10. Thereafter, the tips 22a of the piston rods 22 reach the space V1 side beyond the projection 10c.

Next, the microneedle member 30 is attached to the piston body 20. Specifically, the projections 31b of the microneedle member 30 are engaged with the grooves 21b of the piston body 20, so as to integrate the microneedle member 30 and the piston body 20 with each other, thereby constructing the piston P.

Upon reaching the space VI, the tips 22a of the piston rods 22 regain their original forms and thus engage (catch) the projection 10c of the tubular body 10. Consequently, as illustrated in Fig. 5(a), the piston P is secured to the tubular body 10 against the biasing force of the conical coil spring 40. Therefore, the projection 10c of the tubular body 10 and the tips 22a of the piston rods 22 can be regarded as securing means for securing the piston P to the tubular body 10. Securing the piston P to the tubular body 10 is also referred to as cocking. In this embodiment, the metal wire constituting the conical coil spring 40 has no overlapping part as seen in the center line direction of the conical coil spring 40, whereby the conical coil spring 40 held between the piston plate 21 and the partition wall 10a in the state where the piston P is secured (cocked) to the tubular body 10 has a height on a par with its wire diameter (see Fig. 5(a)).

When performing the cocking in a state where the conical coil spring 40 is completely compressed and storing the puncture device 1 in this state, the conical coil spring 40 is more likely to fatigue (worsen its mechanical properties) than when performing the cocking in a state where the conical coil spring 40 is not completely compressed and storing the puncture device 1 in this state. It is therefore preferred for the conical coil spring 40 to be stored in the puncture device 1 without being completely compressed. Hence, in view of the occurrence of fatigue, the cocking may be performed in a state where the conical coil spring 40 having a higher strength is not completely compressed, such that the piston P attains a desirable velocity when the cocking is released.

Next, the ring member 11 is secured to an end part on the space V1 side of the tubular body 10. Consequently, when released from the securing (cocking) to the tubular body 10, the piston P abuts against the bottom wall part 11b of the ring member 11 and thus is prevented from jumping out of the tubular body 10.

Subsequently, the cap 50 is arranged within the space V2 of the tubular body 10 such that the projection 51 and depression 52 face the through hole 10b. Then, the ring member 12 is secured to an end part on the space V2 side of the tubular body 10. The top wall part 12b of the ring member 12 prevents the cap 50 from jumping out of the tubular body 10.

Since the position of the cap 50 is not fixed within the space V2, the cap 50 can move freely along the extending direction of the housing H (tubular body 10) within the space V2. Therefore, the depression 52 of the cap 50 comes into contact with the tips 22a of the piston rods 22. Thus, this embodiment needs no presser spring and the like for pressing the cap 50, whereby the number of components can be cut down.

The puncture device 1 is manufactured through the foregoing steps. Hence, the conical coil spring 40 keeps its compressed state until the puncture device 1 is used by a user after its shipment subsequent to the manufacture.

[Method of Using Puncture Device]

A method of using the puncture device 1 will now be explained. First, at a location where a medicament or the like is to be applied on the skin, the puncture device 1 is positioned such that the microneedles 32 face the skin. While holding the puncture device 1 in this state, the cap 50 is pushed.

When the cap 50 is pushed, the mortar-shaped depression 52 abuts against the tips 22a of the piston rods 22 and deflects the tips 22a toward the center of the piston plate 21. When the tips 22a deflect such as to be able to pass through the through hole 10b, the engagement between the tips 22a and the projection 10c of the tubular body 10 is released. As a result, the piston P is released from the securing (cocking) to the tubular body 10 and is moved to the outside of the tubular body 10 (to the skin) under the biasing force of the conical coil spring 40, whereby the microneedle member 30 collides with the skin.

When the microneedle member 30 collides with the skin, the microneedles 32 pierces the skin. The velocity of the microneedles 32 (piston P) at this time may be 4 m/s to 30 m/s, 4 m/s to 15 m/s, or 7 m/s to 15 m/s. When the microneedles 32 are configured such as to collide with the skin at a velocity of 4 m/s to 30 m/s, the microneedles 32 can appropriately pierce the skin, thereby allowing the medicament or the like to transfer sufficiently into the animal body.

As in the foregoing, the puncture device 1 pierces the skin when the user simply pushes the cap 50. Therefore, no matter who uses the puncture device 1, the biasing force of the conical coil spring 40 is transmitted to the microneedles 32 through the piston P, so that the microneedles 32 pierce the skin with a fixed impulse force, whereby the skin can securely be pierced (the puncture reproducibility is enhanced). When the microneedles 32 pierce the skin, the active ingredient of the coating C attached to the microneedles 32 is administered into the body, so as to transfer into the body through the skin.

When the microneedle member 30 collides with the skin, the buffer member 23 attached to the piston plate 21 comes into contact with the buffer member 13 attached to the ring member 11. This can reduce the collision sound occurring when the actuated piston P stops at the ring member 11.

[Operations and Effects]

The foregoing embodiment uses the conical coil spring 40 for applying a biasing force to the piston P. When compressed, the conical coil spring 40 is much shorter in height than typical cylindrical coil springs. This can reduce the height of the puncture device 1 itself, thereby making it lighter in weight. Therefore, appropriately designing the conical coil spring 40 can improve the portability of the puncture device 1 while achieving a desirable transfer ratio (ratio of the amount of the medicament or the like transferred into the animal body to the amount of the coating C applied onto the base 31 and/or microneedles 32).

Depending on the kind of medicament and the like, the puncture device must be held for a long time on the skin after colliding therewith. Even in such a case, the puncture device 1 in accordance with this embodiment made smaller in size and lighter in weight enables its user to wear clothes and move without limitation while keeping the puncture device 1 attached to the skin. The puncture device 1 in accordance with this embodiment is small in size and thus is very unlikely to collide with other objects (obstacles) and let the microneedles 32 come out of the skin or break and remain in the skin even when the user behaves freely as such.

The conventional large puncture devices may take time to handle or intimidate users because of large exterior sizes. By contrast, the puncture device in accordance with this embodiment made smaller in size and lighter in weight can easily be handled and greatly reduce the fear that might be felt by the users.

For piercing the skin with the microneedles when the piston body and the microneedle member are separate from each other, the microneedle member is initially arranged on the skin, and then the puncture device is placed on the microneedle member, so as to let the piston body collide with the microneedle member. Unless both the microneedle member and puncture device are arranged appropriately in this case, a positional deviation may occur therebetween, thus hindering the microneedles from piercing the skin appropriately, thereby failing to transfer the medicament and the like sufficiently into the animal body. In the puncture device 1 in accordance with this embodiment, by contrast, a plurality of microneedles 32 project from a main surface of the piston P (surface of the base 31). This enables the medicament or the like to transfer securely into the animal body without such a fear, whereby the puncture device 1 can provide performances as intended by its manufacturer. In addition, simply putting the puncture device 1 on the skin completes its setting on the skin, whereby the setting time is very short.

If the microneedle member is bonded to the piston body with an adhesive and the like so as to integrate them together, organic compounds contained in the adhesive may affect the medicament and the like applied to the tips of microneedles. In this embodiment, by contrast, the piston P comprises the piston body 20 having the piston plate 21 formed with the grooves 21b and the microneedle member 30 having the base 31 provided with the projections 31b adapted to engage the grooves 21b, and the projections 31b engage the grooves 21b, so as to integrate the microneedle member 30 and the piston body 20 with each other. This does not affect the medicament and the like and enables them to provide their intrinsic effects.

In this embodiment, the metal wire constituting the conical coil spring 40 has no overlapping part as seen in the extending direction of the center line of the conical coil spring 40. Therefore, when a load is applied to the conical coil spring 40 along the extending direction of the center line, the height of compressed conical coil spring 40 substantially coincides with its wire diameter. This makes the puncture device 1 further smaller in size and lighter in weight.

[Other Embodiments]

Though preferred embodiment of the present invention are explained in detail in the foregoing, the present invention is not limited to the above-mentioned embodiment. For example, the microneedle member 30 and the piston body 20 are integrated with each other by the engagement between the projections 31b and grooves 21b in the above-mentioned embodiment, but may also be integrated with each other by bonding the microneedle member 30 to the other main surface of the piston body 20 with an adhesive or a bonding sheet, or the other main surface of the piston body 20 may integrally be formed with the microneedles 32.

The microneedles 32, which are arranged at substantially equally spaced intervals in a zigzag (staggered) pattern on the surface of the base 31 in this embodiment, may have different heights on the base 31. For example, the microneedles 32 may have a higher density near the center of the base 31 than on the periphery side or vice versa.

The microneedles 32 may have the same height or different heights. When the microneedles 32 have different heights, they may be higher near the center of the base than on the periphery side or vice versa.

As illustrated in Fig. 9(b), a conical coil spring 41 having both end parts cut flat so as to extend along a virtual plane orthogonal to the center line may be used. End parts on the smaller and larger diameter sides of the conical coil spring 41 abut against the partition wall 10a and piston plate 21, respectively. Therefore, thus constructing the conical coil spring 41 increases the contact areas between the conical coil spring 41 and the partition wall 10a and piston plate 21. Hence, the conical coil spring 41 can be arranged stably within the tubular body 10.

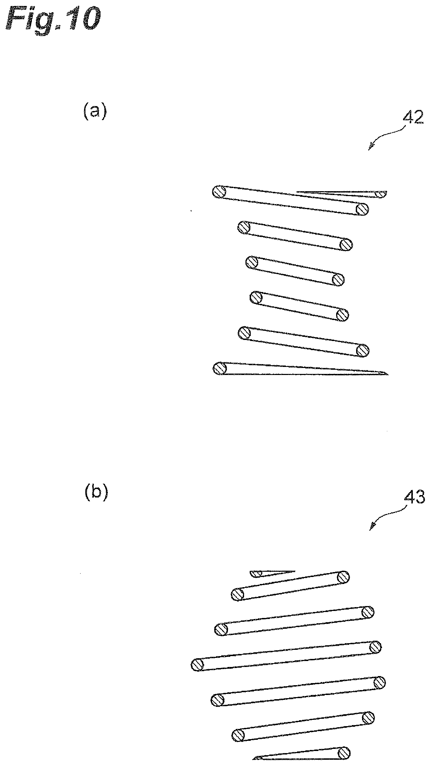

While the conical coil spring 40 is used for applying a biasing force to the piston P in this embodiment, nonlinear coil springs in other forms may also be used. Examples of the nonlinear coil springs in other forms include an hourglass-shaped coil spring 42 (see Fig. 10(a)) and a barrel-shaped coil spring 43 (see Fig. 10(b)).

While, in this embodiment, the metal wire constituting the conical coil spring 40 has no overlapping part as seen in the extending direction of the center line of the conical coil spring 40, the conical coil spring 40 whose metal wire is wound such as to have an overlapping part as seen in the extending direction of the center line may be used. In either case, the free height h of the conical coil spring 40 can be set such as to become smaller than a value obtained by multiplying the wire diameter d by the total number of turns.

The piston body 20 and the microneedle member 30, which are integrated with each other in this embodiment, may be separate from each other. When they are separate from each other, the microneedle member (microneedle array) is placed on the skin, the puncture device 1 is mounted on the skin so as to face the microneedle member, and then the puncture device 1 is actuated, whereby the piston body 20 collides with the microneedle member on the skin, thus piercing the skin.

While the microneedle member 30 is integrated with the piston body 20 in the above-mentioned embodiment, the lower face of the piston body 20 may integrally be formed with the microneedles 32. In this case, the piston body 20 can be treated like the substrate of the microneedle member. That is, the microneedle member can be seen to behave as the piston plate 20.

**Examples**

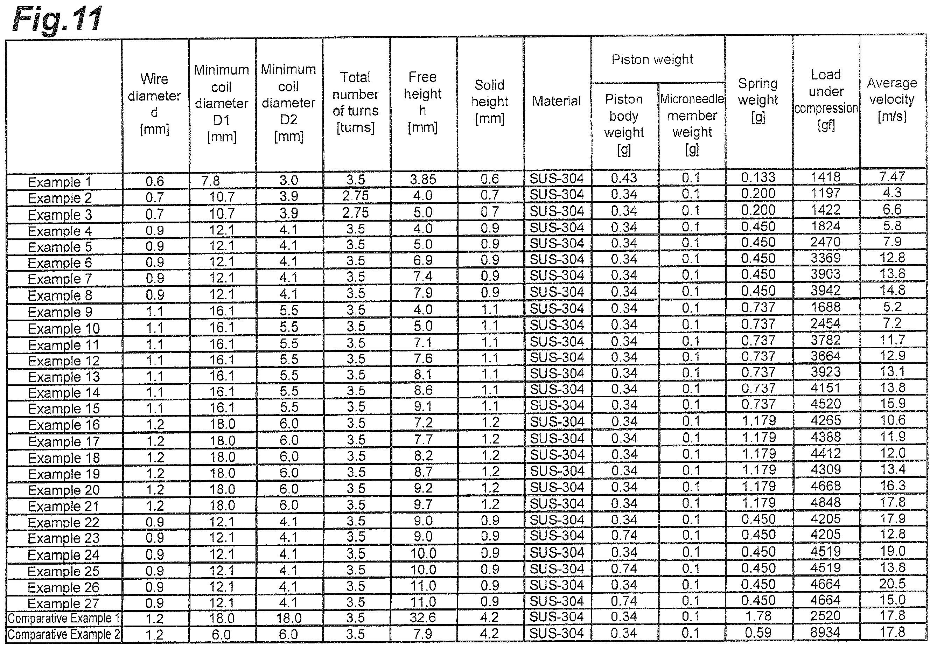

The present invention will now be explained more specifically with reference to Examples 1 to 21 and Comparative Examples and Fig. 11, but are not limited to the following Examples.

(Examples 1 to 27)

Puncture devices in accordance with Examples 1 to 27 in which various parameters (wire diameter, maximum diameter, minimum diameter, total number of turns, free height, solid height, material, and weight) of conical coil springs and piston weight (piston body weight and microneedle member weight) were designed according to Fig. 11 were prepared. For each of the puncture devices in accordance with Examples 1 to 27, the cap was pushed so as to release the piston from being secured, the velocity of the microneedles at the time of colliding with the skin was measured three times, and an average velocity was obtained. A load was exerted on each of the conical coil springs used in the puncture devices in accordance with Examples 1 to 27, and the magnitude of load at the time when the conical coil spring became flat (i.e., at the time when the height of the conical coil spring was substantially the same as the wire diameter) was measured as the load under compression.

(Comparative Example 1)

In Comparative Example 1, parameters (wire diameter, total number of turns, solid height, and material) of a cylindrical coil were made identical to those of the conical coil spring in Example 21, the piston weight was made identical to that of Example 21, the diameter of the cylindrical coil spring was set to 18 mm, and then the free height h of the cylindrical coil spring yielding the same velocity as that of Example 21 was calculated as follows:

First, the spring constant k of the cylindrical coil spring was calculated as k = 869.8 N/m according to the following expression (1):
[Math. 1]

$$k = \frac{Gd^4}{8nD^3} \quad \cdot \cdot \cdot \quad (1)$$

where

G [N/m] is the modulus of transverse elasticity ($68500$ N/mm$^2$ in the case of SUS-304);
d [m] is the wire diameter (the same as Example 21 and thus is 1.2 mm);
n is the total number of turns (the same as Example 21 and thus is 3.5); and
D is the diameter (set to 18 mm in Comparative Example 1).

Subsequently, the spring weight m of the cylindrical coil spring was calculated as m = 1.78 g according to the following expression (2):
[Math. 2]

$$m = \frac{p\pi L d^2}{4} \quad \cdot \cdot \cdot \quad (2)$$

where

p [kg/m$^3$] is the density (7.93 g/cm$^3$ in the case of SUS-304); and
L [m] is the length of the cylindrical coil spring when stretched into a line, which is determined by diameter D $\times$ pi $\times$ total number of turns n.

Then, the energy E of the cylindrical coil spring was calculated as E = 0.35 kg·m$^2$/s$^2$ according to the following expression (3):
[Math. 3]

$$E = \frac{1}{2} M_{total} \cdot V^2 \quad \cdot \cdot \cdot \quad (3)$$

where

$M_{total}$ [kg] is the total of the piston weight and spring weight; and
V [m/s] is the velocity at the time when the microneedles collide with the skin (the same as Example 21 and thus is 17.8 m/s).

Next, the amount of deflection x of the cylindrical coil spring was calculated as x = 28.4 mm according to the following expression (4):
[Math. 4]

$$x = \sqrt{\frac{2E}{k}} \quad \cdot \cdot \cdot \quad (4)$$

Then, the total compressed length of the cylindrical coil spring (= wire diameter d $\times$ total number of turns n) was added to the obtained value x, whereby the free height h was calculated as h = 32.6 mm.

(Comparative Example 2)

In Comparative Example 2, parameters (wire diameter, total number of turns, solid height, and material) of a cylindrical coil were made identical to those of the conical coil spring in Example 21, the piston weight was made identical to that of Example 21, the diameter of the cylindrical coil spring was set to 6 mm, and then the free height h of the cylindrical coil spring yielding the same velocity as that of Example 21 was calculated in the same procedure as with Comparative Example 1. As a result, the free height h was calculated as h = 7.9 mm in Comparative Example 2.

(Evaluation Results)

While the microneedles can appropriately pierce the skin and thereby fully transfer the medicament and the like into the animal body when they are constructed such as to collide with the skin at a velocity of 4 m/s to 30 m/s, all of Examples 1 to 27 are seen to fall within this range. When Example 21 is compared with Comparative Examples 1 and 2, it is seen that, for attaining the same velocity, Comparative Example 1 increases the free height, solid height, and spring weight, while Comparative Example 2 increases the solid height. Hence, Example 21 is seen to achieve smaller size and lighter weight than Comparative Examples 1 and 2.

For the load under compression, when the conical coil springs having the same wire diameter are compared with each other, it is seen that the velocity tends to increase with the load under compression, so that there is a substantially proportional relationship between the load under compression and the velocity. When the conical coil springs having different wire diameters are compared with each other at the same load under compression, it is seen that the velocity tends to increase with the wire diameter.

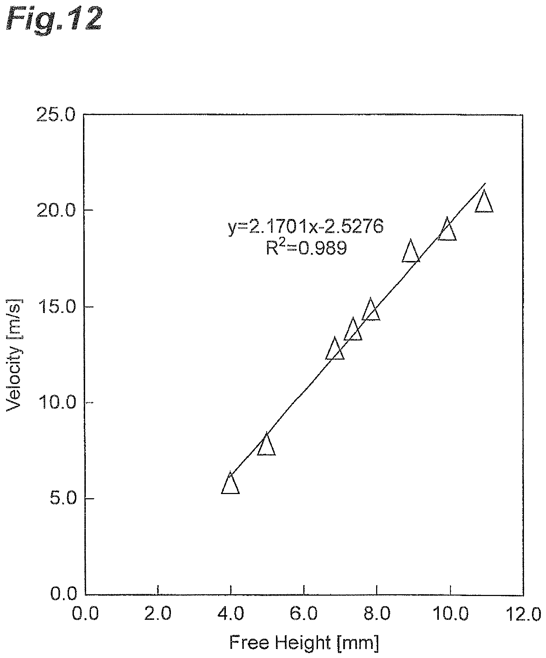

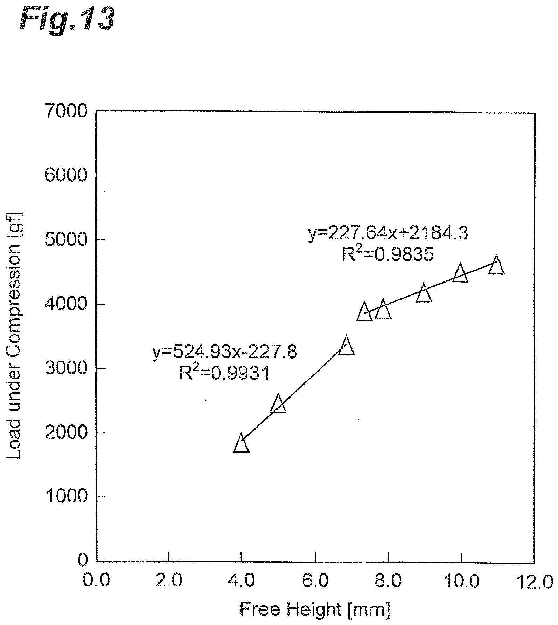

For Examples 4 to 8, 22, 23, 24, 25, 26, and 27, Fig. 12 illustrates the relationship between free height and average velocity, while Fig. 13 illustrates the relationship between free height and load under compression. When the free height is greater than a certain level, as illustrated in Figs. 12 and 13, the velocity of colliding with the skin increases in proportion to the free height, while the increase rate of the load under compression becomes smaller in a region where the free height is large. That is, it is seen that the collision velocity can be raised with a smaller amount of increase in load under compression when the free height of the conical coil spring is 7.4 mm or greater. When the load under compression is large, greater loads act on the piston and housing, thereby making it necessary for the piston and housing to enhance their mechanical strength, which may increase the size and weight of the device as a whole. However, it is seen that making the conical coil spring 40 have a certain free height or higher suppresses the load under compression and reduces loads on the piston and housing while increasing the velocity.

(Examples 28 to 30)

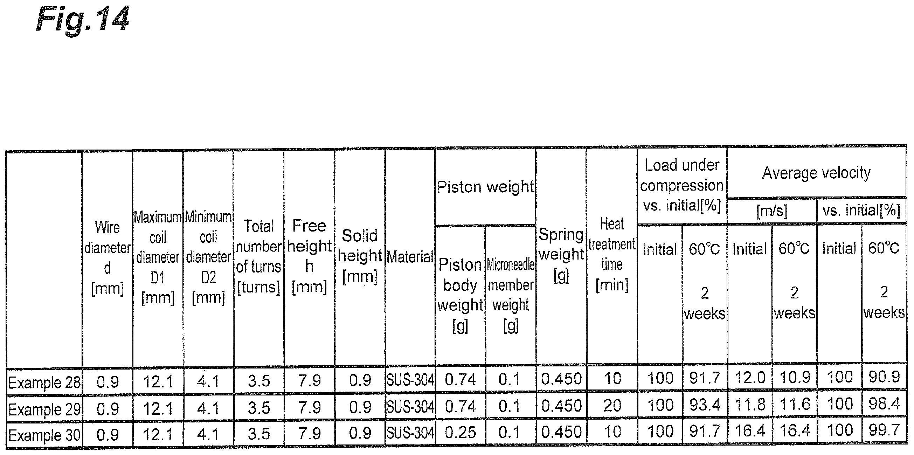

Puncture devices in accordance with Examples 28 to 30 in which various parameters (wire diameter, maximum diameter, minimum diameter, total number of turns, free height, solid height, material, weight, and heat treatment time during manufacture) of conical coil springs and piston weight (piston body weight and microneedle member weight) were designed according to Fig. 14 were prepared. In Examples 28 to 30, the velocity and load under compression were measured in each of the conical coil springs before (as initial one) and after storage for 2 weeks in an environment at 60°C in a flat state (where the conical coil spring was compressed such that its height was substantially the same as the wire diameter), so as to evaluate the durability of the conical coil spring when the heat and load acted on the conical coil spring. Specifically, for each of the puncture devices in accordance with Examples 28 to 30, the cap was pushed so as to release the piston from being secured, the velocity of the microneedles at the time of colliding with the skin was measured three times, and an average velocity was obtained. A load was exerted on each of the conical coil springs used in the puncture devices in accordance with Examples 28 to 30, and the magnitude of load at the time when the conical coil spring became flat (i.e., at the time when the height of the conical coil spring was substantially the same as the wire diameter) was measured as the load under compression.

(Evaluation Results)

As can be understood from Examples 28 and 29, the velocity and load under compression are harder to deteriorate as the heat treatment time of the conical coil spring is longer, whereby the durability of the conical coil spring is seen to improve.

As can be understood from Examples 28 and 30, by reducing the weight of the piston body, the velocity is seen to be harder to deteriorate in the conical coil springs having the same load under compression. That is, it is seen that the durability of collision velocity in the conical coil spring can be improved when the ratio of the weight of the piston body to the total weight of the piston body, microneedle member, and conical coil spring is made smaller. With regard to this discovery and according to the claimed invention, the ratio of the weight of the piston body to the total weight of the piston body, microneedle member, and conical coil spring is less than 50%.

**Reference Signs List**

1: puncture device; 10: housing; 20: piston body; 21: piston plate; 21b: groove; 30: microneedle member; 31: base; 31b: projection; 32: microneedle; 40: conical coil spring; 50: cap; P: piston

**Claims**

1. A puncture device (1) for transferring an active ingredient into a body through a skin by piercing the skin with a microneedle (32), the puncture device comprising:

    a tubular housing (H);
    a piston (P) having a piston body (20) which is slidably arranged within the housing and has a main surface intersecting a sliding direction, and a plurality of microneedles which are arranged on the main surface side; and
    a conical coil spring (40) arranged within the housing and providing the piston with a biasing force,
    wherein the ratio of the weight of the piston body to the total weight of the piston body, the plurality of microneedles, and the conical coil spring is less than 50%.

2. A puncture device according to claim 1, wherein the piston has a piston body formed with a groove (21b) and a microneedle member (30) provided with an engagement piece (31b) adapted to engage the groove; and
    wherein the microneedle member includes a surface constituting the main surface of the piston, the plurality of microneedles projecting from the surface.

3. A puncture device according to claim 1 or 2, wherein the conical coil spring is formed by a stainless steel wire, a piano wire, or a copper wire.

4. A puncture device according to any one of claims 1 to 3, wherein the conical coil spring has a free height (h) of at least three times a wire diameter (d) thereof.

5. A puncture device according to any one of claims 1 to 4, wherein the conical coil spring has a free height of at least 1 mm to 100 mm.

6. A puncture device according to any one of claims 1 to 5, wherein a metal wire constituting the conical coil spring has no overlapping part as seen in an extending direction of a center line of the conical coil spring.

7. A puncture device according to any one of claims 1 to 6, wherein the conical coil spring has both end parts cut flat along a virtual plane orthogonal to the center line of the conical coil spring.

8. A puncture device according to any one of claims 1 to 7, wherein the conical coil spring has a maximum diameter (D1) of 1 mm to 100 mm.

9. A puncture device according to any one of claims 1 to 8, wherein the conical coil spring has a minimum diameter (D2) of at least 1/1000 but less than 1 times the maximum diameter thereof.

10. A puncture device according to any one of claims 1 to 9, wherein the conical coil spring has a wire diameter (d) of 0.01 mm to 2 mm.

11. A puncture device according to any one of claims 1 to 10, wherein the conical coil spring has a load of 10,8 N (1100 gf) to 49 N (5000 gf) under compression.

**Patentansprüche**

1. Punktionsvorrichtung (1) zum Übertragen eines Wirkstoffs in einen Körper durch eine Haut durch Durchstechen der Haut mit einer Mikronadel (32), wobei die Punktionsvorrichtung umfasst:

ein rohrförmiges Gehäuse (H);
einen Kolben (P) mit einem Kolbenkörper (20), der verschiebbar innerhalb des Gehäuses angeordnet ist und eine Hauptoberfläche, die eine Gleitrichtung durchschneidet, und eine Vielzahl von Mikronadeln aufweist, die auf der Hauptoberflächenseite angeordnet sind; und
eine konische Schraubenfeder (40), die innerhalb des Gehäuses angeordnet ist und den Kolben mit einer Vorspannkraft versorgt,
wobei das Verhältnis des Gewichts des Kolbenkörpers zum Gesamtgewicht des Kolbenkörpers, der Vielzahl von Mikronadeln und der konischen Schraubenfeder weniger als 50% beträgt.

2. Punktionsvorrichtung nach Anspruch 1, wobei der Kolben einen mit einer Nut (21b) ausgebildeten Kolbenkörper und ein Mikronadelelement (30) aufweist, das mit einem Eingriffsstück (31b) versehen ist, das zum Einrasten in die Nut geeignet ist; und
wobei das Mikronadelelement eine Oberfläche beinhaltet, die die Hauptoberfläche des Kolbens bildet, wobei die Vielzahl von Mikronadeln von der Oberfläche vorsteht.

3. Punktionsvorrichtung nach Anspruch 1 oder 2, wobei die konische Schraubenfeder durch einen Edelstahldraht, einen Klavierdraht oder einen Kupferdraht gebildet ist.

4. Punktionsvorrichtung nach einem der Ansprüche 1 bis 3, wobei die konische Schraubenfeder eine freie Höhe (h) von mindestens dem Dreifachen eines Drahtdurchmessers (d) davon aufweist.

5. Punktionsvorrichtung nach einem der Ansprüche 1 bis 4, wobei die konische Schraubenfeder eine freie Höhe von mindestens 1 mm bis 100 mm aufweist.

6. Punktionsvorrichtung nach einem der Ansprüche 1 bis 5, wobei ein Metalldraht, der die konische Schraubenfeder bildet, keinen überlappenden Teil aufweist, wie in einer sich ausdehnenden Richtung einer Mittellinie der konischen Schraubenfeder gesehen.

7. Punktionsvorrichtung nach einem der Ansprüche 1 bis 6, wobei die konische Schraubenfeder beide Endteile flach geschnitten entlang einer virtuellen Ebene orthogonal zur Mittellinie der konischen Schraubenfeder aufweist.

8. Punktionsvorrichtung nach einem der Ansprüche 1 bis 7, wobei die konische Schraubenfeder einen maximalen Durchmesser (D1) von 1 mm bis 100 mm aufweist.

9. Punktionsvorrichtung nach einem der Ansprüche 1 bis 8, wobei die konische Schraubenfeder einen minimalen Durchmesser (D2) von mindestens 1/1000, aber weniger als das 1-fache des maximalen Durchmessers davon aufweist.

10. Punktionsvorrichtung nach einem der Ansprüche 1 bis 9, wobei die konische Schraubenfeder einen Drahtdurchmesser von 0,01 mm bis 2 mm aufweist.

**11.** Punktionsvorrichtung nach einem der Ansprüche 1 bis 10, wobei die konische Schraubenfeder eine Last von 10,8 N (1100 gf) bis 49 N (5000 gf) unter Druck aufweist.

## Revendications

**1.** Dispositif pour piqûre (1) destiné à transférer un ingrédient actif dans un corps à travers une peau en perçant la peau avec une microaiguille (32), le dispositif pour piqûre comprenant :

un logement tubulaire (H) ;
un piston (P) ayant un corps de piston (20) qui est disposé de manière coulissante à l'intérieur du logement et présente une surface principale en intersection avec un sens de coulissement, et une pluralité de microaiguilles qui sont disposées sur le côté de surface principale ; et
un ressort de bobine conique (40) disposé à l'intérieur du logement et fournissant au piston une force de sollicitation,
le rapport du poids du corps de piston au poids total du corps de piston, la pluralité des microaiguilles, et le ressort de bobine conique étant inférieur à 50 %.

**2.** Dispositif pour piqûre selon la revendication 1, dans lequel le piston présente un corps de piston formé d'une rainure (21b) et d'un élément de microaiguille (30) muni d'une pièce d'engagement (31b) adaptée pour engager la rainure ; et
l'élément de microaiguille comprenant une surface constituant la surface principale du piston, la pluralité des microaiguilles se projetant depuis la surface.

**3.** Dispositif pour piqûre selon la revendication 1 ou 2, le ressort de bobine conique étant formé d'un fil en acier inoxydable, d'un câble à piano, ou d'un fil de cuivre.

**4.** Dispositif pour piqûre selon l'une quelconque des revendications 1 à 3, dans lequel le ressort de bobine conique présente une hauteur libre (h) d'au moins trois fois son diamètre de fil (d).

**5.** Dispositif pour piqûre selon l'une quelconque des revendications 1 à 4, dans lequel le ressort de bobine conique présente une hauteur libre d'au moins 1 mm à 100 mm.

**6.** Dispositif pour piqûre selon l'une quelconque des revendications 1 à 5, dans lequel un câble métallique constituant le ressort de bobine conique ne présente pas de partie chevauchante tel qu'observé dans un sens d'extension d'une ligne centrale du ressort de bobine conique.

**7.** Dispositif pour piqûre selon l'une quelconque des revendications 1 à 6, dans lequel le ressort de bobine conique possède les deux parties d'extrémité coupées plates le long d'un plan virtuel orthogonal à la ligne centrale du ressort de bobine conique.

**8.** Dispositif pour piqûre selon l'une quelconque des revendications 1 à 7, dans lequel le ressort de bobine conique présente un diamètre maximal (D1) de 1 mm à 100 mm.

**9.** Dispositif pour piqûre selon l'une quelconque des revendications 1 à 8, dans lequel le ressort de bobine conique présente un diamètre minimum (D2) d'au moins 1/1 000 mais inférieur à 1 fois son diamètre maximal.

**10.** Dispositif pour piqûre selon l'une quelconque des revendications 1 à 9, dans lequel le ressort de bobine conique présente un diamètre de fil (d) de 0,01 mm à 2 mm.

**11.** Dispositif pour piqûre selon l'une quelconque des revendications 1 à 10, dans lequel le ressort de bobine conique présente une charge de 10,8 N (1 100 gf) à 49 N (5 000 gf) sous compression.

16

*Fig.1*

EP 2 835 147 B1

*Fig.2*

18

*Fig.3*

*Fig.4*

50

51

52

## *Fig.5*

(a)

(b)

Fig.6

EP 2 835 147 B1

Fig.7

# Fig.8

(a)

(b)

(c)

*Fig.9*

(a)

(b)

*Fig.10*

(a)

42

(b)

43

## Fig.11

| | Wire diameter d [mm] | Minimum coil diameter D1 [mm] | Minimum coil diameter D2 [mm] | Total number of turns [turns] | Free height h [mm] | Solid height [mm] | Material | Piston weight | | Spring weight [g] | Load under compression [gf] | Average velocity [m/s] |
|---|---|---|---|---|---|---|---|---|---|---|---|---|
| | | | | | | | | Piston body weight [g] | Microneedle member weight [g] | | | |
| Example 1 | 0.6 | 7.8 | 3.0 | 3.5 | 3.85 | 0.6 | SUS-304 | 0.43 | 0.1 | 0.133 | 1418 | 7.47 |
| Example 2 | 0.7 | 10.7 | 3.9 | 2.75 | 4.0 | 0.7 | SUS-304 | 0.34 | 0.1 | 0.200 | 1197 | 4.3 |
| Example 3 | 0.7 | 10.7 | 3.9 | 2.75 | 5.0 | 0.7 | SUS-304 | 0.34 | 0.1 | 0.200 | 1422 | 6.6 |
| Example 4 | 0.9 | 12.1 | 4.1 | 3.5 | 4.0 | 0.9 | SUS-304 | 0.34 | 0.1 | 0.450 | 1824 | 5.8 |
| Example 5 | 0.9 | 12.1 | 4.1 | 3.5 | 5.0 | 0.9 | SUS-304 | 0.34 | 0.1 | 0.450 | 2470 | 7.9 |
| Example 6 | 0.9 | 12.1 | 4.1 | 3.5 | 6.9 | 0.9 | SUS-304 | 0.34 | 0.1 | 0.450 | 3369 | 12.8 |
| Example 7 | 0.9 | 12.1 | 4.1 | 3.5 | 7.4 | 0.9 | SUS-304 | 0.34 | 0.1 | 0.450 | 3903 | 13.8 |
| Example 8 | 0.9 | 12.1 | 4.1 | 3.5 | 7.9 | 0.9 | SUS-304 | 0.34 | 0.1 | 0.450 | 3942 | 14.8 |
| Example 9 | 1.1 | 16.1 | 5.5 | 3.5 | 4.0 | 1.1 | SUS-304 | 0.34 | 0.1 | 0.737 | 1688 | 5.2 |
| Example 10 | 1.1 | 16.1 | 5.5 | 3.5 | 5.0 | 1.1 | SUS-304 | 0.34 | 0.1 | 0.737 | 2454 | 7.2 |
| Example 11 | 1.1 | 16.1 | 5.5 | 3.5 | 7.1 | 1.1 | SUS-304 | 0.34 | 0.1 | 0.737 | 3782 | 11.7 |
| Example 12 | 1.1 | 16.1 | 5.5 | 3.5 | 7.6 | 1.1 | SUS-304 | 0.34 | 0.1 | 0.737 | 3664 | 12.9 |
| Example 13 | 1.1 | 16.1 | 5.5 | 3.5 | 8.1 | 1.1 | SUS-304 | 0.34 | 0.1 | 0.737 | 3923 | 13.1 |
| Example 14 | 1.1 | 16.1 | 5.5 | 3.5 | 8.6 | 1.1 | SUS-304 | 0.34 | 0.1 | 0.737 | 4151 | 13.8 |
| Example 15 | 1.1 | 16.1 | 5.5 | 3.5 | 9.1 | 1.1 | SUS-304 | 0.34 | 0.1 | 0.737 | 4520 | 15.9 |
| Example 16 | 1.2 | 18.0 | 6.0 | 3.5 | 7.2 | 1.2 | SUS-304 | 0.34 | 0.1 | 1.179 | 4265 | 10.6 |
| Example 17 | 1.2 | 18.0 | 6.0 | 3.5 | 7.7 | 1.2 | SUS-304 | 0.34 | 0.1 | 1.179 | 4388 | 11.9 |
| Example 18 | 1.2 | 18.0 | 6.0 | 3.5 | 8.2 | 1.2 | SUS-304 | 0.34 | 0.1 | 1.179 | 4412 | 12.0 |
| Example 19 | 1.2 | 18.0 | 6.0 | 3.5 | 8.7 | 1.2 | SUS-304 | 0.34 | 0.1 | 1.179 | 4309 | 13.4 |
| Example 20 | 1.2 | 18.0 | 6.0 | 3.5 | 9.2 | 1.2 | SUS-304 | 0.34 | 0.1 | 1.179 | 4668 | 16.3 |
| Example 21 | 1.2 | 18.0 | 6.0 | 3.5 | 9.7 | 1.2 | SUS-304 | 0.34 | 0.1 | 1.179 | 4848 | 17.8 |
| Example 22 | 0.9 | 12.1 | 4.1 | 3.5 | 9.0 | 0.9 | SUS-304 | 0.34 | 0.1 | 0.450 | 4205 | 17.9 |
| Example 23 | 0.9 | 12.1 | 4.1 | 3.5 | 9.0 | 0.9 | SUS-304 | 0.74 | 0.1 | 0.450 | 4205 | 12.8 |
| Example 24 | 0.9 | 12.1 | 4.1 | 3.5 | 10.0 | 0.9 | SUS-304 | 0.34 | 0.1 | 0.450 | 4519 | 19.0 |
| Example 25 | 0.9 | 12.1 | 4.1 | 3.5 | 10.0 | 0.9 | SUS-304 | 0.74 | 0.1 | 0.450 | 4519 | 13.8 |
| Example 26 | 0.9 | 12.1 | 4.1 | 3.5 | 11.0 | 0.9 | SUS-304 | 0.34 | 0.1 | 0.450 | 4664 | 20.5 |
| Example 27 | 0.9 | 12.1 | 4.1 | 3.5 | 11.0 | 0.9 | SUS-304 | 0.74 | 0.1 | 0.450 | 4664 | 15.0 |
| Comparative Example 1 | 1.2 | 18.0 | 18.0 | 3.5 | 32.6 | 4.2 | SUS-304 | 0.34 | 0.1 | 1.78 | 2520 | 17.8 |
| Comparative Example 2 | 1.2 | 6.0 | 6.0 | 3.5 | 7.9 | 4.2 | SUS-304 | 0.34 | 0.1 | 0.59 | 8934 | 17.8 |

## Fig.12

$y=2.1701x-2.5276$
$R^2=0.989$

*Fig.13*

## Fig.14

| | Wire diameter d [mm] | Maximum coil diameter D1 [mm] | Minimum coil diameter D2 [mm] | Total number of turns [turns] | Free height h [mm] | Solid height [mm] | Material | Piston weight | | Spring weight [g] | Heat treatment time [min] | Load under compression vs. initial[%] | | Average velocity | | | |
|---|---|---|---|---|---|---|---|---|---|---|---|---|---|---|---|---|---|
| | | | | | | | | | | | | | | [m/s] | | vs. initial[%] | |
| | | | | | | | | Piston body weight [g] | Microneedle member weight [g] | | | Initial | 60°C 2 weeks | Initial | 60°C 2 weeks | Initial | 60°C 2 weeks |
| Example 28 | 0.9 | 12.1 | 4.1 | 3.5 | 7.9 | 0.9 | SUS-304 | 0.74 | 0.1 | 0.450 | 10 | 100 | 91.7 | 12.0 | 10.9 | 100 | 90.9 |
| Example 29 | 0.9 | 12.1 | 4.1 | 3.5 | 7.9 | 0.9 | SUS-304 | 0.74 | 0.1 | 0.450 | 20 | 100 | 93.4 | 11.8 | 11.6 | 100 | 98.4 |
| Example 30 | 0.9 | 12.1 | 4.1 | 3.5 | 7.9 | 0.9 | SUS-304 | 0.25 | 0.1 | 0.450 | 10 | 100 | 91.7 | 16.4 | 16.4 | 100 | 99.7 |

EP 2 835 147 B1

**EP 2 835 147 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4659332 B
- JP 2007516781 A
- WO 2009107806 A
- WO 00009184 A
- US 2011276027 A
- WO 2004009172 A1